# EUROPÄISCHE PATENTANMELDUNG

(11) **EP 4 403 030 A1**
(43) Veröffentlichungstag der Anmeldung: **24.07.2024**
(21) Anmeldenummer: 23152339.0
(22) Anmeldetag: 19.01.2023
(51) Int. Cl.: A21B 1/40, G01N 33/10

(54) **VERFAHREN ZUR ÜBERWACHUNG DER GARE EINES TEIGS IN EINEM GARRAUM**

(71) Anmelder: Bake the Shape GmbH, 4481 Asten (AT)
(72) Erfinder: Rauch, Gerald, 5730 Mittersill (AT); Danninger, Thomas, 4063 Hörsching (AT)
(74) Vertreter: Schwarz & Partner Patentanwälte GmbH

(57) **Zusammenfassung**

Die vorliegende Erfindung betrifft ein Verfahren zur Kontrolle der Gare eines Teigs für Backwaren, wobei der Teig in einem Garraum (1) über einen gewissen Zeitraum unter gewissen Garparametern bis hin zu einem Gar-Ende gegart wird, dadurch gekennzeichnet, dass in den Garraum (1) mindestens eine Probe des Teigs in mindestens einem Referenzbehälter (2) eingebracht wird und an der mindestens einen Probe zumindest eine für die Gare relevante Probeneigenschaft gemessen wird.

## Beschreibung

Die vorliegende Erfindung betrifft ein Verfahren und eine Vorrichtung zur Kontrolle der Gare von Gargut im Bereich von Backwaren in einem Garraum.

### Hintergrund der Erfindung

In industriellen Gärprozessen im Bereich von Backwaren ist der Schritt der Gare qualitätsbestimmend und damit entscheidend für die Wirtschaftlichkeit eines Verfahrens. Bei falsch eingestellter Gare steigt der Ausschuss je Backzyklus auf bis zu 15% da die Produkte nicht mehr den Qualitätskriterien der Hersteller entsprechen.

Da nachfolgende Prozesse die eventuell entstandenen Produktfehler auf Grund von mangelnder Garequalität nur in sehr beschränktem Ausmaß beeinflussen können, ist eine Überwachung der Gare für den weiteren Prozessverlauf essenziell.

Bei industriellen Gärprozessen ist oft eine "knappe Gare" erwünscht. Dies bedeutet, dass der Teigling eine ausgeprägte Form und ein ausreichend großes Volumen aufweist. Der Teigling ist locker, elastisch und zeigt bei leichtem Abtasten etwas Widerstand. Während der knappen Gare hat der Teigling eine bestimmte Gärreife erreicht, um in den Ofen eingeschoben werden zu können. In diesem Stadium ist die Gasbildung im Teigling noch gering. Dies bewirkt, dass fertig gebackene Backwaren eine feine Porung mit einer elastischen, saftigen und aromatischen Krume bilden.

Die Teiglinge befinden sich in der knappen Gare oder auch "3/4 Gare" am Anfang der sogenannten Gärtoleranz. Der Begriff "3/4 Gare" bedeutet, dass der Teigling seine volle Gare noch nicht erreicht hat. Die restliche Gare erreicht er im sogenannten Ofentrieb. Dieser setzt unmittelbar nach dem Einschießen der Teiglinge in den Ofen ein.

Für die Zwecke der vorliegenden Erfindung wird der gewünschte Grad der Gare, bevor die Teiglinge in den Offen eingebracht werden, als "Gar-Ende" bezeichnet.

Im Falle einer Untergare kann die Verlängerung der Garzeit, sowie eine Erhöhung der Ofentemperatur und der Verweilzeit im Ofen zu einer verbesserten Produktqualität führen.

Für den Fall der Übergare kann ein Verkürzen der Backzeit unter Umständen eine Abhilfemaßnahme sein.

Wenn unterschiedliche Teige in den Garraum eingebracht werden, ist bei gleichbleibenden Bedingungen im Garraum wie Temperatur und Luftfeuchtigkeit das Erreichen des Gar-Endes zu unterschiedlichen Zeitpunkten möglich. Hier ist eine Steuerung allein durch Messen der Bedingungen im Garraum schwierig.

Weiters wäre für manche Eigenschaften des zu garenden Teigs wie z.B. Temperatur und Feuchtigkeit eine invasive Messung (z.B. mit Sensoren innerhalb des Teigs selbst) wünschenswert. Dies führt aber zu einer Beschädigung des Teigs.

Die oben dargestellten Probleme gelten umso mehr für in Backformen befindliche Teiglinge. Backformenteile bestehen oft aus zwei Formhälften, die durch einen Schließmechanismus zusammengehalten werden, wie z.B. in WO2013/116884A1 beschrieben. In diesem Fall ist eine präzise Bestimmung der Garverhältnisse derzeit überhaupt nicht möglich. Die Systeme sind während des gesamten Prozessdurchlaufs immer wieder in Bewegung versetzt und können daher nicht fix mit einer Sensorik verbunden werden.

Eine weitere Problematik betrifft das Auftreten von Produktfehlern aufgrund einer unzureichenden Gareüberwachung.

Insbesondere bei einer Vielzahl von Teiglingen, welche beispielsweise in Backformen aus zwei Formhälften gegart werden, führen Produktfehler zu einem hohen Ausschuss an Backware je nach Prozesszyklus. Typische Produktfehler sind beispielsweise gekennzeichnet durch eine ungleichmäßige Porung des Teigs, Teigaustritt zwischen den Backformhälften sowie einer mangelhaften Formausbildung des Teiges in der Backform und einer mäßig gelockerten Krume mit zu dichter Porung.

Dem Fachmann sind vielfältige Verfahren zum Steuern und zur Überwachung von Garprozessen von Lebensmitteln aller Art bekannt. Dieser Umstand liegt bereits darin begründet, dass häufig in Abhängigkeit von der Gargutart sowie der Menge des Garguts unterschiedliche Garverläufe zu jeweils optimalen Resultaten führen.

Aus der DE 102019107846 A1 ist beispielsweise ein Verfahren bekannt, bei dem die Größe des Garguts während des Garprozesses mittels einer Kameraeinrichtung überwacht wird. Die ermittelte Gargutgröße wird mit einem Referenzwert verglichen, sodass der Garzustand eines Lebensmittels, bevorzugt Fleisch, Gemüse oder Obst, bestimmt wird. Die Ermittlung weiterer relevanter Probeneigenschaften ist jedoch nicht vorgesehen.

Die DE 102019107815 B4 offenbart ein Verfahren zum Betreiben eines Gargeräts, wobei die Temperatur des Garguts während des Garprozesses einerseits durch eine Messsonde auf der Oberfläche des Garguts ermittelt wird und anderseits die ausgehende Wärmestrahlung des Garguts erfasst wird. Aus der Temperaturdifferenz wird die Bräunung des Garguts ermittelt.

Aus der DE 102019101695 A1 ist ein Verfahren zum Garen von Gargut bekannt, bei dem das Gargut durch eine Wärmebildkamera erfasst wird und die eingetragene Hochfrequenzstrahlung in den Garraum bei abweichender Wärmeverteilung auf dem Gargut durch eine Steuereinrichtung entsprechend verändert wird.

Die DE 102008010099 B4 beschreibt ein Verfahren zur Bestimmung des Zustands eines in einem Garraum befindlichen Garguts aus der Abhängigkeit der ermittelten Strom/Spannungs-Charakteristik, die während des Garens auftreten. Die Strom/Spannungs-Charakteristik sowie das Gasentladungs- und Glimmentladungsspektrum werden herangezogen, um Information über den Zustand der Gare eines Garguts zu erhalten.

Ferner offenbart die EP 3282818 A1 ein Verfahren und eine Vorrichtung zum Erhitzen eines Garguts in einem Garraum mittels eines Hochfrequenzerzeugers. Dadurch wird das Gargut durch die Leistung der Hochfrequenzstrahlung gezielt oberflächennah erhitzt und gebräunt, wobei die Überwachung der Gare durch das Gargerät keine Beachtung findet.

Schließlich ist aus der EP 2101229 B2 ein Verfahren zum Bereitstellen eines intelligenten Mensch-Maschinen-Interfaces bei Gargeräten bekannt, in dem der Anwender durch getätigte Handlungen am Gargerät in eine oder mehrere Anwenderklassen eingeordnet wird. Durch die Klassifizierung der Anwender wird automatisch eine personalisierte Modifizierung von Parametern von Arbeitsprogrammen hervorgerufen. Eine Überwachung des Garguts während der Garprozesse oder eine Auswertung der Garungsparameter ist jedoch nicht vorgesehen.

Keines der vorstehend zitierten Dokumente des Standes der Technik beschäftigt sich mit der Überwachung der Gare von Teigen von Backwaren.

### Zusammenfassung der Erfindung

Der vorliegenden Erfindung liegt die Aufgabe zugrunde, ein verbessertes Verfahren zur Überwachung des Garprozesses von Teigen für Backwaren bereitzustellen.

Die Aufgabe ist gelöst durch ein Verfahren zur Kontrolle der Gare eines Teigs für Backwaren, wobei der Teig in einem Garraum über einen gewissen Zeitraum unter gewissen Garparametern bis hin zu einem Gar-Ende gegart wird, dadurch gekennzeichnet, dass in den Garraum mindestens eine Probe des Teigs in mindestens einem Referenzbehälter eingebracht wird und an der mindestens einen Probe zumindest eine für die Gare relevante Probeneigenschaft gemessen wird.

Ein weiterer Aspekt der vorliegenden Erfindung betrifft einen Referenzbehälter zur Durchführung eines Verfahrens, enthaltend
- einen Sensor zur Temperaturermittlung innerhalb einer Probe des Teigs
- einen Lasersensor zur Volumensermittlung einer Probe des Teigs und
- einen kapazitiven Sensor zur Ermittlung der korrekten Positionierung des Referenzbehälters.

### Kurzbeschreibung der Figuren

Fig. 1 zeigt den Garraum 1 inklusive Referenzbehälter 2. Die Vorrichtung der vorliegenden Erfindung ist im Garraum 1 an einem fixen Ort montiert, sodass für jeden Referenzbehälter 2 gewährleistet ist, dass immer an derselben Stelle gemessen wird. Der dazugehörende Stikkenwagen 7 befindet sich dabei direkt hinter dem Referenzbehälter 2, womit eine eindeutige Zuordnung von Stikkenwagen 7 zu Referenzbehälter 2 gegeben ist.
Fig. 2 zeigt den Garraum 1 inklusive mehrerer Referenzbehälter 2 pro Stikkenwagen 7. Je ein Stikkenwagen 7 befindet sich dabei direkt hinter einem Referenzbehälter 2, womit eine eindeutige Zuordnung von Stikkenwagen 7 zu Referenzbehälter 2 gegeben ist.
Fig. 3a zeigt den Referenzbehälter 2 gemäß der vorliegenden Erfindung in Frontansicht, umfassend einen Temperatursensor 3, einen Lasersensor 4 und einen kapazitativen Sensor 5.
Fig. 3b zeigt eine Seitenansicht des Referenzbehälters 2, umfassend einen Temperatursensor 3, einen Lasersensor 4 und einen kapazitativen Sensor 5.
Fig. 3c zeigt eine schematische Darstellung des Referenzbehälters 2 gemäß der vorliegenden Erfindung.

### Beschreibung der Ausführungsformen

Die vorliegende Erfindung betrifft ein Verfahren zur Kontrolle der Gare eines Teiges für Backwaren. Unter dem Begriff "Backware" ist ein Lebensmittel zu verstehen, das auf der Basis von Getreide und/oder Getreideerzeugnissen nach Zugabe von Wasser oder wasserhaltigen Flüssigkeiten sowie von anderen Lebensmitteln in der Regel durch Kneten, Formen, Lockern und Backen oder Kochextrudieren eines Teiges und Backen desselben hergestellt wird.

Ein ungebackener, portionierter und vorgeformter Teig wird im Allgemeinen als "Teigling" beschrieben. In weiterer Folge soll unter dem Begriff "Teig" sowohl ein ungebackener und ungeformter als auch ein ungebackener, portionierter und vorgeformter Teig verstanden werden.

Es hat sich überraschenderweise gezeigt, dass mit der Erhebung von Probeneigenschaften in einem Referenzbehälter die ideale Garedauer und der Zeitpunkt bis zum Backen im Ofen präzise bestimmt werden können. Der vorliegenden Erfindung liegt dabei die Erkenntnis zugrunde, dass Rückschlüsse auf die Zusammenhänge des Gesamtprozesses im Zusammenspiel mit den gemessenen Probeneigenschaften mindestens einer Probe genau jenes Teigs, der auch gegart wird, gezogen werden können.

Dadurch kann ein optimiertes Garverfahren erreicht werden, bei dem der Fortschritt der Gare kontinuierlich überwacht und bereits im Vorfeld Parameterabschätzungen für den Gareprozess getroffen werden können.

Als Garparameter können bevorzugt die Temperatur und/oder Feuchte im Garraum und/oder die Dauer der Gare ausgewählt werden.

Die Messung der Probeneigenschaft an der Probe des Teigs erfolgt typischerweise über denselben Zeitraum, in welchem sich der zu garende Teig im Garraum befindet.

In einer besonders vorteilhaften Ausführungsform des Verfahrens werden als Probeneigenschaft die Temperatur der Probe, das Volumen der Probe, die Feuchtigkeit der Probe und gegebenenfalls mehrere dieser Eigenschaften gemessen.

Insbesondere anhand der Erhebung der Daten für diese Eigenschaften in der Probe im Referenzbehälter kann der ideale Zeitpunkt bis zum "Einschießen in den Ofen" präzise bestimmt werden.

Im Folgenden steht der Begriff "Probeneigenschaft" (in Einzahl), soweit nicht näher konkretisiert, stellvertretend für sowohl eine als auch gegebenenfalls mehrere gemessene Probeneigenschaft(en).

Bevorzugt wird die Probeneigenschaft durch mindestens einen Sensor und/oder mindestens eine Infrarotkamera ermittelt.

Gemäß einer weiteren Ausführungsform ist das Verfahren dadurch gekennzeichnet, dass die Temperatur der mindestens einen Probe des Teigs durch einen Temperatursensor ermittelt wird, wobei der Sensor zur Messung der Temperatur in die Probe eingeführt wird. Die Temperatur kann dabei diskret oder kontinuierlich überwacht werden.

In dieser Ausführungsform zeigt sich ein besonderer Vorteil des erfindungsgemäßen Verfahrens, da das Einbringen eines Temperatursensors in einen Teig nicht ohne Beschädigung des Teigs bzw. im Fall einer Backform gar nicht möglich wäre.

Eine besonders bevorzugte Ausführungsform des Verfahrens ist dadurch gekennzeichnet, dass das Volumen der mindestens einen Probe des Teigs durch einen Lasersensor ermittelt wird. Die Volumenbestimmung erfolgt mittels diskreter oder kontinuierlicher Laservermessung der Teigoberfläche in vertikaler Ausdehnungsrichtung. Über die Änderung der Füllhöhe des Referenzbehälters mit dem Teig wird das Volumen der Probe während des Gärprozesses bestimmt.

Bis zum Vorliegen einer Kennlinie kann das Gar-Ende manuell, über das Erreichen eines bestimmten Volumens und/oder einer bestimmten Temperatur, eingestellt werden.

Eine "Kennlinie" ist eine graphische Darstellung des Zusammenhangs zwischen den ermittelten Werten einer Probeneigenschaft und einer definierten Garzeit. Anhand der Kennlinien für das Volumen und/oder die Temperatur der mindestens einen Probe des Teigs kann das Gar-Ende abgeschätzt werden.

Der Begriff "Gar-Ende" bezieht sich, wie oben erwähnt, auf den Zeitpunkt, bei dem der Teig die gewünschte Gärreife erreicht hat, um in den Ofen eingeschoben werden zu können.

Eine besonders bevorzugte Ausführungsform des Verfahrens ist dadurch gekennzeichnet, dass das Gar-Ende über das Erreichen eines bestimmten Volumens und/oder einer bestimmten Temperatur der mindestens einen Probe des Teigs festgestellt wird.

In einer weiteren bevorzugten Ausführungsform ist das Verfahren der vorliegenden Erfindung dadurch gekennzeichnet, dass der mindestens eine, die Probe enthaltende Referenzbehälter in die Nähe des zu garenden Teigs eingebracht wird.

Um die Gareüberwachung zu standardisieren und abzusichern, können im Garraum fixe Positionierungen für die Referenzbehälter vorgegeben werden. Bevorzugt wird der Referenzbehälter über einen Hydraulikzylinder von der Decke des Garraums in die Nähe des Teigs oder des Behälters für den zu garenden Teig eingebracht. Sobald der Stikkenwagen an seinem Platz im Garraum steht, wird der Referenzbehälter nach unten befördert.

Unter "Stikkenwagen" ist ein fahrbares Gestell für die Aufnahme und den Transport von Teigen oder für Behälter für zu garende Teige zu verstehen, das vollständig in einem dafür passenden Ofen geschoben werden kann. In einen auf Rollen laufenden Stikkenwagen können übereinander Teige oder Behälter mit dem zu garenden Teig eingeschoben werden, sodass der Ofen in einem Arbeitsgang be- und entladen werden kann.

In einer besonders vorteilhaften Ausführungsform des Verfahrens erfolgt die Ermittlung der korrekten Positionierung des mindestens einen Referenzbehälters durch einen Sensor, insbesondere durch einen kapazitiven Sensor.

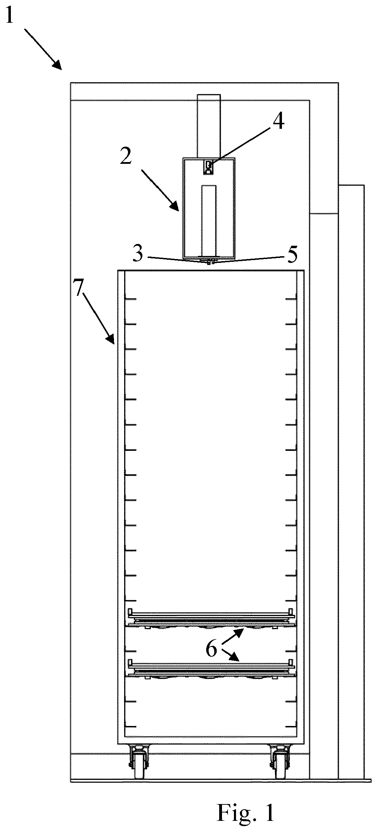

Fig. 1 zeigt, dass der mindestens eine Referenzbehälter 2 im Garraum 1 an einem fixen Ort montiert werden kann, sodass für jeden Referenzbehälter 2 gewährleistet ist, dass immer an derselben Stelle gemessen wird. Der dazugehörende Stikkenwagen 7 befindet sich dabei in der Nähe des Referenzbehälters 2, womit eine eindeutige Zuordnung von Stikkenwagen 7 zu Referenzbehälter 2 gegeben ist. Zusätzlich kann die Zugehörigkeit zwischen Referenzbehälter 2 und Stikkenwagen 7 noch mit RFID-Chips abgesichert werden.

Ist die Gare abgeschlossen, kann der Referenzbehälter 2 wieder zur Decke des Garraums 1 befördert werden und der Stikkenwagen 7 kann aus dem Garraum 1 hinausgefahren und in den Ofen eingeschlossen werden.

Wie sich aus dem Obigen ergibt, kann mit dem erfindungsgemäßen Verfahren die Gare verschiedener Teige durch Messung der Probeneigenschaft jeweils einer Probe des jeweiligen Teiges überwacht werden. Sobald für den betreffenden Teig anhand der gemessenen Probeneigenschaften das Gar-Ende erreicht wurde, kann der Teig (bzw. der den Teig führende Stikkenwagen) aus dem Garraum entfernt werden, während für andere Teige die Gare weiterläuft.

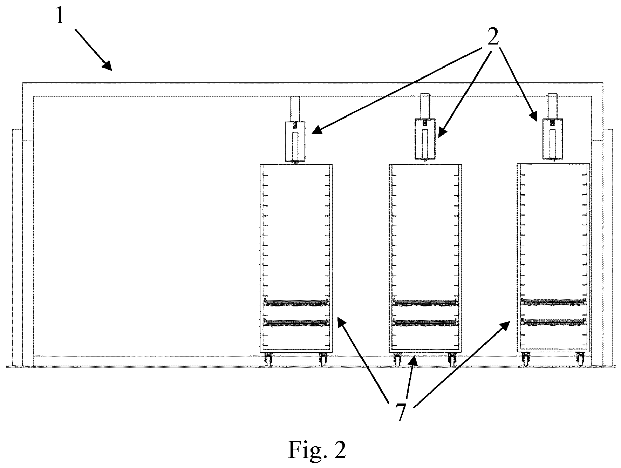

Fig. 2 zeigt, dass die Gare von verschiedenen Teigen, welche sich je in einem Referenzbehälter 2 im Garraum 1 befinden, gemessen werden kann. Jeweils eine Probe pro Stikkenwagen 7 wird in jeweils einen Referenzbehälter 2 eingebracht. Der dazugehörende Stikkenwagen 7 befindet sich dabei in der Nähe des Referenzbehälters 2, womit eine eindeutige Zuordnung von Stikkenwagen 7 zu Referenzbehälter 2 gegeben ist.

Das erfindungsgemäße Verfahren eignet sich insbesondere für Backverfahren, bei denen der zu garende Teig in einem geschlossenen Behälter, insbesondere in einer geschlossenen Backform gegart wird.

Mehrere Backformen können zu einem Backformenverbund zusammengefasst sein. Ein Backformenverbund kann Backformen unterschiedlicher Größe und Form umfassen.

Eine Vielzahl von mit Teig gefüllten Backformen kann in Form von Backformenverbunden auf einem Stikkenwagen in den Garraum eingebracht werden. Eine Überwachung der Gare von einer Vielzahl an Teigen in Backformen war bislang äußerst schwierig.

In dieser Ausführungsform des erfindungsgemäßen Verfahrens kann bevorzugt die Temperatur von mindestens einem Behälter für den zu garenden Teig durch eine Infrarotkamera ermittelt werden.

Die Erfassung der Backformenverbundtemperatur erfolgt durch eine Infrarotkamera, welche in der Nähe des Stikkenwagens angebracht ist. Vorzugsweise wird die Backformenverbundtemperatur vor dem Einbringen der Verbunde in den Garraum gemessen.

In einer weiteren Ausführungsform ist das Verfahren gemäß der vorliegenden Erfindung dadurch gekennzeichnet, dass die Garparameter anhand der gemessenen Probeneigenschaft gesteuert werden.

Eine Steuerung kann dabei punktuell (d.h. anhand der diskreten Messung der Probeneigenschaft zu einem gewissen Zeitpunkt) erfolgen. Ebenso möglich ist eine kontinuierliche Steuerung.

Die Temperatur und/oder das Volumen der mindestens einen Probe des Teigs, und/oder die Temperatur des mindestens einen Behälters für den zu garenden Teig können diskret oder kontinuierlich aufgezeichnet werden. Hier kann ein vorbestimmtes Toleranzband vorgegeben werden, in dem sich die Temperatur und/oder das Volumen idealerweise bewegen soll. Positive oder negative Abweichungen zu den Sollwerten dienen zur Steuerung der Garezeit. Als zusätzliche Kontrolle können die Temperaturen und/oder das Volumen auf einer Anzeigeeinheit direkt angezeigt werden, wobei die betroffenen Stikkenwägen mit einer Markierung versehen werden können, die anzeigt, dass die Garezeit angepasst werden muss. Der betroffene Stikkenwagen kann daher entweder schon vor oder nach dem ursprünglich anvisierten Gar-Ende aus dem Gareraum befördert werden.

Vorzugsweise wird mindestens ein Garparameter gesteuert, um das Volumen und/oder die Temperatur der mindestens einen Probe des Teigs und/oder die Temperatur des mindestens einen Behälters für den zu garenden Teig zu beeinflussen. Liegt ein Istwert einer Probeneigenschaft außerhalb eines vorbestimmtes Toleranzbandes, kann mindestens ein Garparameter derart gesteuert werden, sodass der Istwert der Probeeigenschaft wieder in diesem Toleranzband liegt.

Eine Steuerung der Garparameter kann manuell oder automatisiert erfolgen.

Vorzugsweise wird der Schwellenwert des Minimalvolumens und/oder des Maximalvolumens der mindestens einen Probe des Teigs anhand der ermittelten Temperatur der mindestens einen Probe des Teigs und/oder des mindestens einen Behälters für den zu garenden Teig verändert. Im Fall einer Veränderung der Temperatur durch tageszeitliche Schwankung der Umgebungsbedingungen oder Abweichungen in der Kühlvorrichtung, kann das zu erreichende Volumen anhand einer hinterlegten Kennlinie angepasst werden. Dadurch kann sichergestellt werden, dass die Garparameter an die Temperatur gekoppelt sind.

In einer weiteren Ausführungsform der vorliegenden Erfindung wird ein Minimalvolumen und/oder ein Maximalvolumen mit wenigstens einem hinterlegten Schwellenwert verglichen und anhand des Vergleichs das Gar-Ende ermittelt. In dieser Ausführungsform wird das Erreichen eines Volumens oberhalb des Schwellenwerts für das Minimalvolumen und/oder unterhalb des Schwellenwerts für das Maximalvolumen als eine Voraussetzung für das Erreichen des Gar-Endes definiert.

In einer weiteren bevorzugten Ausführungsform werden durch die Daten der ermittelten Probeneigenschaften Kennfelder erstellt und aufgrund der Kennfelder der aktuelle Garverlauf mit den Kennfeldern verglichen.

Es ist also eine kontinuierliche Datensammlung vorgesehen, die es ermöglicht, den ablaufenden Gärprozess präzise zu analysieren. Im Zusammenspiel mit den ermittelten Daten aus der Garüberwachung können Rückschlüsse auf die Zusammenhänge des Gesamtprozesses gezogen werden. Die gemessenen Werte können über den zeitlichen Verlauf in ein Diagramm eingetragen und über den Kurvenverlauf können Aussagen über den Fortschritt der Gare abgeleitet werden.

Die Auswertungen sind dabei in Echtzeit abrufbar und die jeweiligen Verläufe den einzelnen Produktionschargen zuordenbar.

Ein weiterer Vorteil betrifft die ermittelten Kennfelder für verschiedene Versuchsbackreihen, welche für unterschiedliche Produkte unter Serienbedingungen erstellt werden können. Die ermittelten Kennfelder können zwecks Veranschaulichung des Gärprozesses zur Dokumentation der Garparameter als auch zur Beurteilung der daraus resultierenden gemessenen Probeneigenschaften verwendet werden. Die ermittelten Kennfelder stellen eine hoch verdichtete Informationsquelle dar, aus der eine Beurteilung der vorliegenden Prozessbedingungen abgeleitet werden kann.

Mit den Erkenntnissen aus den Messreihen und dem Erstellen von Kennfeldern können bereits im Vorfeld Parameterabschätzungen für den Gare- und Backprozess getroffen werden.

Durch diese Vorgangsweise können die Anfahrausschusszahlen reduziert und insgesamt die Anzahl der Backreihen bis zu einem qualitativ optimierten Produkt gesenkt werden. Durch eine umfangreiche Datensammlung ist es auch möglich, die Durchlaufzeiten zu optimieren, da über den Anstieg der Volumenkurve auch Aussagen über den tatsächlichen Reifezustand des Teigs getroffen werden können.

Die Datenaufzeichnung und Aufbereitung können über geeignete Software erfolgen. Mittels der Software können dann nutzerspezifische Auswertungen erstellt werden und in regelmäßigen Abständen als Kennzahlen zur Prozesssteuerung und Kontrolle herangezogen werden. Durch die Vernetzung der Daten aus den unterschiedlichen Prozessschritten können die qualitätsrelevanten Prozesse präzise überwacht und geregelt werden. Anhand der gesammelten Daten können mittels der Software-Auswertungen auch die Einflüsse von tageszeitlichen und jahreszeitlichen Schwankungen ermittelt werden.

Mit diesen Erkenntnissen werden einerseits die für die Technologie relevanten Parameter wissenschaftlich aufbereitet, und andererseits können mit den vorliegenden Informationen die Produktionsparameter auf veränderte Umgebungsbedingungen angepasst werden.

Vorzugsweise wird beim Erreichen des Gar-Endes ein optisches und/oder ein akustisches Signal ausgelöst. Dadurch kann einer zu langen Gare und damit einem Qualitätsverlust des Teigs vorgebeugt werden, da der Teig aus dem Garraum rechtzeitig hinausbefördert und in den Ofen eingeschossen werden kann.

Ein weiterer Aspekt der vorliegenden Erfindung betrifft einen Referenzbehälter zur Durchführung eines Verfahrens.

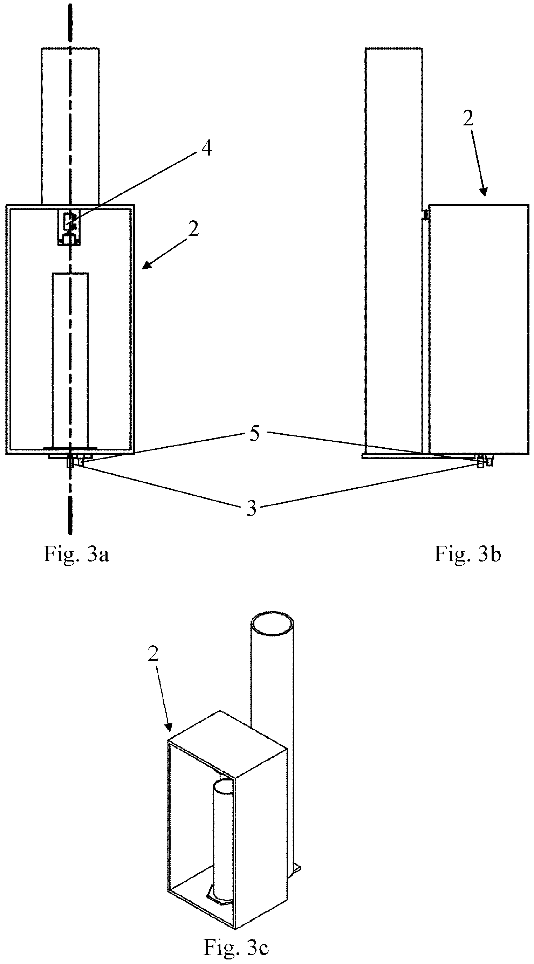

Fig. 3a, 3b und 3c zeigen, dass der vorliegende Referenzbehälter 2 ein definiertes Volumen besitzt, um die für die Gare relevante Probeneigenschaften zu messen. Dazu umfasst der Behälter einen Lasersensor 4, einen Temperatursensor 3 und einen bevorzugt kapazitiven Sensor 5. Dabei ist der Lasersensor 4 in der Mitte des Referenzbehälters 2 positioniert. Über eine Bohrung am Boden in der Mitte des Zylinders kann der Temperatursensor 3 in den Teig eingeführt werden.

Gemäß einer Ausführungsform der vorliegenden Erfindung ist der Referenzbehälter dadurch gekennzeichnet, dass der mindestens eine Referenzbehälter eine Bohrung am Boden aufweist und dass der Sensor zur Temperaturermittlung durch die Bohrung zur Außenseite des Referenzbehälters hindurchführt.

### Beispiele

### Beispiel 1:

Eine vorgeformte Teigmenge wird in Backformen eingebacht, welche zu Backformenverbunden zusammengeschlossen werden. Die befüllten Backformen bzw. Backformenverbunde werden in Stikkenwägen eingeschlichtet und in den Garraum befördert. Die Stikkenwägen werden an ihren zugewiesenen Positionen im Garraum platziert, sodass jeder Stikkenwagen unter einem Referenzbehälter steht. Die Referenzbehälter werden über Hydraulikzylinder von der Decke des Garraums herabgelassen und jeweils eine Probe der Teigmasse eines Stikkenwagens in einem Referenzbehälter eingebracht.

Anschließend wird das Garende manuell über das Erreichen eines bestimmten Volumens der Proben eingestellt. Während des Garprozesses werden Temperatur und Volumen jeder der Proben in den Referenzbehältern gemessen und aufgezeichnet. Nach dem Erreichen des gewünschten Volumens einer Probe (nach ca. 40 bis 60 Minuten Garzeit) wird der betreffende Referenzbehälter wieder zur Decke des Garraums hochgefahren und der jeweilige Stikkenwagen in den Ofen transportiert, in dem der Teig für ca. 20 bis 30 Minuten gebacken wird.

### Beispiel 2:

Eine über eine Teigformanlage vorgeformte Teigmenge wird über ein Förderband direkt in die Backformen eingebracht, welche wiederum zu Backformenverbunden zusammengeschlossen werden. Während des Vorbeifahrens der Backformenverbunde an einer Infrarotkamera werden die Temperaturen der Backformenverbunde umfassend der Backformen gemessen, wobei die Infrarotkamera stationär über dem Förderband positioniert ist. Aus diesen Temperaturen kann eine voraussichtliche Gardauer ermittelt werden, die aber in weiterer Folge anhand der tatsächlichen Entwicklung der Gare angepasst werden kann.

Die Backformverbunde werden anschließend in Stikkenwägen geschlichtet und die ermittelten Temperaturwerte der Backformenverbunde werden den einzelnen Stikkenwägen zugeordnet. Die Stikkenwägen werden in den Garraum transferiert und an ihren zugewiesenen Positionen platziert. Jeweils eine Probe der Teigmasse pro Stikkenwagen wird in jeweils einen Referenzbehälter eingebracht, wie in Beispiel 1 beschrieben.

Vor Beginn der Gare wird das Garende über das Erreichen eines bestimmten Volumens pro Probe manuell eingestellt. Die ermittelten Temperaturen der Backformenverbunde der einzelnen Stikkenwägen werden mit vorher ermittelten Kennlinien in Korrelation gebracht und damit das zu erreichende Volumen pro Probe automatisch angepasst. Temperatur und Volumen jeder in einem Referenzbehälter befindlichen Probe werden während der Gare kontinuierlich gemessen und aufgezeichnet. Die aufgezeichneten Probeneigenschaften werden während des Garprozesses mittels einer Prozesssoftware (Microsoft Power BI und Azure Datawarehouse) in Korrelation gebracht und damit die Garzeit der Probe individuell, auf Basis dieser Daten, modifiziert.

Zusätzlich werden die ermittelten Probeneigenschaften während des Garprozess mit Kennlinien verglichen und durch entsprechende Steuerung der Garparameter angepasst. Nach Erreichen des gewünschten Volumens einer Probe wird der Referenzbehälter dieser Probe entfernt und der dazugehörige Stikkenwagen in den Ofen befördert, in dem der Teig gebacken wird.

## Patentansprüche

1. Verfahren zur Kontrolle der Gare eines Teigs für Backwaren, wobei der Teig in einem Garraum (1) über einen gewissen Zeitraum unter gewissen Garparametern bis hin zu einem Gar-Ende gegart wird, **dadurch gekennzeichnet, dass** in den Garraum (1) mindestens eine Probe des Teigs in mindestens einem Referenzbehälter (2) eingebracht wird und an der mindestens einen Probe zumindest eine für die Gare relevante Probeneigenschaft gemessen wird.

2. Verfahren gemäß Anspruch 1, **dadurch gekennzeichnet, dass** als Probeneigenschaft die Temperatur der Probe, das Volumen der Probe, die Feuchtigkeit der Probe und gegebenenfalls mehrere dieser Eigenschaften gemessen werden.

3. Verfahren gemäß Anspruch 1 oder 2, **dadurch gekennzeichnet, dass** die Probeneigenschaft durch mindestens einen Sensor und/oder mindestens eine Infrarotkamera ermittelt wird.

4. Verfahren gemäß einem der vorhergehenden Ansprüche, **dadurch gekennzeichnet, dass** die Temperatur der mindestens einen Probe des Teigs durch einen Temperatursensor (3) ermittelt wird, wobei der Sensor zur Messung der Temperatur (3) in die Probe eingeführt wird.

5. Verfahren gemäß einem der vorhergehenden Ansprüche, **dadurch gekennzeichnet, dass** das Volumen der mindestens einen Probe des Teigs durch einen Lasersensor (4) ermittelt wird.

6. Verfahren gemäß einem der vorhergehenden Ansprüche, **dadurch gekennzeichnet, dass** das Gar-Ende über das Erreichen eines bestimmten Volumens und/oder einer bestimmten Temperatur der mindestens einen Probe des Teigs festgestellt wird.

7. Verfahren gemäß einem der vorhergehenden Ansprüche, **dadurch gekennzeichnet, dass** der mindestens eine die Probe enthaltende Referenzbehälter (2) in die Nähe des zu garenden Teigs eingebracht wird.

8. Verfahren gemäß einem der vorhergehenden Ansprüche, **dadurch gekennzeichnet, dass** eine Ermittlung der korrekten Positionierung des mindestens einen Referenzbehälters (2) durch einen Sensor (5), insbesondere durch einen kapazitiven Sensor (5), erfolgt.

9. Verfahren gemäß einem der vorhergehenden Ansprüche, **dadurch gekennzeichnet, dass** der zu garende Teig in einem geschlossenen Behälter (6), insbesondere in einer geschlossenen Backform gegart wird.

10. Verfahren gemäß einem der vorhergehenden Ansprüche, **dadurch gekennzeichnet, dass** die Temperatur von mindestens einem Behälter (6) für den zu garenden Teig durch eine Infrarotkamera ermittelt wird.

11. Verfahren gemäß einem der vorhergehenden Ansprüche, **dadurch gekennzeichnet, dass** die Garparameter anhand der gemessenen Probeneigenschaft gesteuert werden.

12. Verfahren gemäß Anspruch 11, **dadurch gekennzeichnet, dass** ein Minimalvolumen und/oder ein Maximalvolumen mit wenigstens einem hinterlegten Schwellenwert verglichen wird und dass anhand des Vergleichs das Gar-Ende ermittelt wird und dass das Erreichen eines Volumens oberhalb des Schwellenwerts für das Minimalvolumen und/oder unterhalb des Schwellenwerts für das Maximalvolumen eine Voraussetzung für das Erreichen des Gar-Endes festgestellt wird.

13. Verfahren gemäß einem der Ansprüche 9 bis 12, **dadurch gekennzeichnet, dass** der Schwellenwert des Minimalvolumens und/oder des Maximalvolumens der mindestens einen Probe des Teigs anhand der ermittelten Temperatur des mindestens einen Behälters (6) für den zu garenden Teig verändert wird.

14. Verfahren gemäß einem der vorangehenden Ansprüche, **dadurch gekennzeichnet, dass** durch die Daten der ermittelten Probeneigenschaft Kennfelder erstellt werden und aufgrund der Kennfelder der aktuelle Garverlauf mit den Kennfeldern verglichen wird.

15. Verfahren gemäß einem der vorangehenden Ansprüche, **dadurch gekennzeichnet, dass** beim Erreichen des Gar-Endes ein optisches und/oder ein akustisches Signal ausgelöst wird.

16. Referenzbehälter (2) zur Durchführung eines Verfahrens gemäß einem der vorhergehenden Ansprüche, enthaltend
- einen Sensor zur Temperaturermittlung (3) innerhalb einer Probe des Teigs
- einen Lasersensor zur Volumensermittlung (4) einer Probe des Teigs und
- einen kapazitiven Sensor (5) zur Ermittlung der korrekten Positionierung des Referenzbehälters.

17. Referenzbehälter (2) gemäß Anspruch 16, **dadurch gekennzeichnet, dass** der mindestens eine Referenzbehälter (2) eine Bohrung am Boden aufweist und dass der Sensor zur Temperaturermittlung (3) durch die Bohrung zur Außenseite des Referenzbehälters (2) hindurchführt.
